# EUROPEAN PATENT SPECIFICATION

(11) **EP 3 448 294 B1**
(45) Date of publication and mention of the grant of the patent: **12.02.2020**
(21) Application number: 17714068.8
(22) Date of filing: 13.03.2017
(51) Int. Cl.: A61B 18/20

(54) **APPLICATOR FOR COOLING SKIN DURING IRRADIATION**
APPLIKATOR ZUR KÜHLUNG DER HAUT WÄHREND DER BESTRAHLUNG
APPLICATEUR POUR REFROIDIR LA PEAU PENDANT L'IRRADIATION

(30) Priority: 26.04.2016 US 201662327509 P; 27.01.2017 US 201762451101 P
(43) Date of publication of application: 06.03.2019
(73) Proprietor: Candela Corporation, Wayland, MA 01778 (US)
(72) Inventor: SCHOMACKER, Kevin, Maynard, MA 01754 (US); BERSTEIN, Jeffrey, Sudbury, MA 01776 (US); GRINIS, Vadim, Natick, MA 01760 (US); CRONIN, Stephen, Norwood, MA 02062 (US); OTTERSON, Herbert, Needham, MA 02492 (US)
(74) Representative: Barker Brettell LLP
(86) International application number: PCT/US2017/022023
(87) International publication number: WO 2017/189109

(56) References cited:
- US-A- 5 595 568
- US-A- 5 849 029
- US-A- 6 096 029
- US-A1- 2005 234 527
- US-A1- 2009 312 693
- US-A1- 2013 178 916
- US-B1- 7 083 610

## Description

### TECHNOLOGY FIELD

The present applicator relates to the field of cosmetic treatment and in particular to cosmetic skin treatment.

### BACKGROUND

Cosmetic skin treatment is typically performed by application of heat to a segment of skin to be treated. The heat could be generated by application of Radio Frequency (RF), Intense Pulse Light (IPL), laser radiation, ultrasound or a combination of the above energies. Almost all of the skin treatment energies are applied to the skin by a tool termed applicator or handpiece. For example, applicators configured to apply to the skin a combination of RF and IPL or laser light systems such as the one disclosed in US 6,702,808, 8,516,706, 9,271,793 to the same assignee and other similar systems. Concurrent application to the skin of RF energy and light energy allows addressing of skin layers located at different depth at the same treatment. Heating of the tissue via the different energies is additive, sometimes the sum being greater than the added parts. The different energies may enter the skin at different locations. The energies applied to the treated skin segment and in particular light energy, are not distributed uniform across the treated skin or tissue segment. Typically the light energy decreases exponentially as the light passes deeper into tissue while in skin, the RF energy decreases laterally as you move away from the electrode. The combination of the two energies helps to better homogenize tissue heating. When the applicator is moved across the skin with a single energy source, some of the areas of the skin segment could be over treated and even burned and some of the areas could be undertreated. Because of these differences use of multiple sources of energy allow reduction of each of the energies and diminish the danger of skin burning.

The adverse effects for example, epidermal injury caused by application of large amount of heat, particularly light, to the treated skin or tissue segment could be somehow mitigated by concurrent cooling of the treated tissue. Cooling of the tissue segment located in a tissue or skin treatment plane is usually performed by cooling an applicator element being in contact with the skin treatment plane. Methods of applying a cooled fluid spray directly to the tissue or skin treatment plane are also known. Cooling with cryogen spray is considered as the most effective epidermis cooling and in particular for the darker skin types.

US patents 6,059,820, 6,530,920, 8,113,209, RE42594, and EP1057454 disclose skin treatment applicators with different skin cooling options. US 2005/234527 A1 discloses a method and apparatus for improving bodily safety during exposure to a monochromatic light source by diverging the monochromatic light with a diffuser. US 7,083,610 B1 discloses a device for irradiating tissue, including a fluorescent element for receiving pump radiation and responsively emitting radiation having different spectral characteristics than the pump radiation.

### SUMMARY

In accordance with the invention there is provided an applicator with a skin cooling arrangement, as defined by the appended claims. Disclosed is an applicator or handpiece configured to apply treatment energy to a segment of skin located in a tissue or skin treatment plane. The treatment energy could be RF energy, applied by a pair of bipolar RF electrodes or optical energy, or a combination of RF and optical energies. The optical energy wavelength could be selected from a number of different optical energy sources as it could be desired by a particular skin treatment.

The applicator or handpiece include a support structure configured to hold a transparent window. The outer surface of the transparent window is in contact with the surface of the skin (dermis) and defines the tissue or skin treatment plane. The transparent window includes one or more fluid conducting channels configured to receive a cooling fluid from a source of cooling fluid and conduct the cooling fluid through the transparent window to cool the tissue or skin surface being in contact with the outer surface of the window. In one example, the contact cooling element is appropriately optimized and is effective for the lighter skin types as the cooling with cryogen sprays is effective for darker skin types. In another example, the contact cooling element and the treatment mode are appropriately optimized to enforce cool down time before delivering the laser energy. Such mode of operation is effective for skin cooling, when treating the darker skin types such as Type Skin V and VI. Applying a contact cooling element to the surface of the skin or tissue prevents non-reversible damage to the dermis.

In one example, the support structure is angled with respect to axis of symmetry of the applicator at an angle of 10 to 30 degrees. The angled with respect to the axis of symmetry support structure gives to operator or caregiver a better view of the tissue treatment plane or area compared to that of an applicator designed to be applied perpendicular to the treatment plane. The support structure includes fluid conducting channels that are in fluid communication with respective fluid conducting channels in the transparent window and conduct the cooling fluid from the source of cooling fluid to and through the transparent window placed in contact with skin. The proximity of the cooled or chilled water with the skin surface reduces the thermal resistance of the chilling or cooling tip. The thermal resistance just under 1 K/W is easily obtained following optimization of the cooling tip design.

In one example, the applicator includes an optical system configured to receive a beam of optical energy from a source of optical energy, which could be a laser or an IPL source and convey or funnel it to the tissue treatment plane. The transparent window terminates the applicator and defines the tissue or skin treatment plane. The beam of optical energy could be conducted from the source of optical energy to the applicator by a fiber optics guide or with the help of an articulated arm. Although the fiber optics guide homogenizes optical energy distribution across the beam of optical energy, the optical system could use a tapered light pipe homogenizing optical energy distribution across the beam of optical energy and a pair of lenses. When an articulated arm is used to conduct the optical energy from the source of optical energy to the applicator, the optical system includes a tapered light pipe homogenizing rod configured to homogenize the cross section of the beam of optical energy and a pair of lenses. The taper can help improve homogenization plus allow smaller diameter optics downstream.

Alternatively, a laser diode or laser diode bar could be located in the applicator and use additional arrangements homogenizing the optical energy distribution across the beam of optical energy.

In one example, the angled support structure of the applicator is configured to support in addition to the transparent window with fluid conducting channels, a pair or more than a pair of the bi-polar RF electrodes. The tissue treatment plane could be located between the pair or pairs of the bipolar RF electrodes.

The optical system is configured to receive the homogenized beam of optical energy from the trapezoidal prism and image the output facet of the trapezoidal prism to a treated skin or tissue plane located between the pair of bipolar RF electrodes. The support structure is angled between 10 to 30 degrees with respect to the tissue treatment plane. The image of the output facet of the trapezoidal prism at the tissue treatment plane could have a round shape, an elliptical/oval shape, a rectangular shape or a shape of a polygon.

In another example, the support structure is not angled with respect to the tissue treatment plane. The support structure is an assembly of fluid conducting tubes or channels. The space between the fluid conducting tubes is sufficient to support unobstructed real time observation or visualization of the tissue treatment plane during cosmetic skin treatment procedures. The support structure is configured to support or hold a transparent window with fluid conducting channels and a pair of bipolar RF electrodes that terminate the applicator.

The tissue treatment plane is located between the bipolar RF electrodes. The skin treatment could be performed by application of RF energy only, optical energy only or a combination of RF energy and optical energy. Each of the applicators could include a cooling arrangement configured to cool the tissue or skin treatment plane to which the treatment energy is applied.

The cooling arrangement could include the transparent window with fluid conducting channels that conduct the cooling fluid flow from the source of cooling fluid or from the support frame. The fluid conducting channels could be configured to conduct the cooling fluid flow to almost the entire surface of the transparent window or at least to a part of the surface to the transparent window. At least one side of the transparent window in course of treatment is in contact with the tissue or skin surface located in the treatment plane and cools the tissue or skin surface located in the treatment plane.

Use of transparent window with the fluid flow conducting channels significantly improves the heat removal capacity by reducing the thermal resistance of the cooling tip and allows expanding the range of power used in the particular skin treatment that could be for example, hair removal. The cooling tip includes the transparent window with fluid flow conducting channels and the support structure and specifically the part of the base of the support structure that is in contact with skin. With proper design of the support structure and the window, thermal resistance of less than 1 degree Celsius per Watt of heat load can be obtained.

The applicator or handpiece significantly improve observation of the tissue or skin treatment plane and facilitate the work of the operator or caregiver, providing unobstructed view of the tissue or skin treatment plane immediately prior to, during, and after the delivery of a treatment pulse without the need of removing the application from the field. The angled support structure and the placement of the homogenizing light pipe more proximal to the tissue treatment plan helps to improve the user's view of the treatment field before, during and after delivering the treatment energy.

### LIST OF FIGURES AND THEIR BRIEF DESCRIPTION

FIG. 1 is a simplified illustration of an applicator disclosed in US 2016/0074672 A1;
FIG. 1A is a view taken along the line C in FIG. 1.
FIG. 2 is a simplified illustration of an applicator according to an example;
FIG, 3A and 3B is an example of a transparent cooling window including cooling fluid flow conducting channels;
FIG. 3A and 3B is an example of a transparent cooling window including cooling fluid flow conducting channels;
FIG. 4A and 4B is an additional example of a transparent cooling window including cooling fluid flow conducting channels;
FIG. 5 is a cross section of transparent cooling window of FIG. 4 along a line indicated by arrows D-D;
FIGS. 6A and 6B is an additional example of a transparent cooling window including cooling fluid flow conducting channels;
FIG. 7A and 7B is still an additional example of an applicator for skin treatment;
FIG. 8A and 8B is still an additional example of a transparent cooling window including cooling fluid flow conducting channels;

### DESCRIPTION

Concurrent application to the tissue or skin located in the treatment plane of RF energy and light energy allows addressing of skin or tissue layers located at different depth at one treatment. It allows reduction of each of the energies and diminishes the danger of skin burning. Appropriate cooling by a contact cooling element applied to the surface of the skin or tissue becomes even more important and is desired to prevent non-reversible damage to the dermis. The present disclosure provides effective contact cooling devices optimized for effective cooling of both the lighter and darker skin types.

The applicator also supports real time observation of visible changes in the treated skin or tissue area and facilitates the accurate placement of the applicator from one application of the skin treatment energy, to the next application of skin treatment energy.

Cooling of the tissue located in a tissue treatment plane is usually performed by cooling an applicator element being in contact with the tissue located in the treatment plane. Absence of effective cooling of the epidermis while heating the dermis and deeper skin layers during the course of optical (laser) energy treatments can cause undesired pain to the treated subject and potentially undesired skin injury. Traditional cooling modes - such as topical applications, forced cold air, and contact cooling - cool imperfectly, are often too long and too deeply. Such methods force additional laser energy to be used to reheat the dermis. Therefore, either treatment fluence needs to be raised or the therapeutic value is diminished.

The authors of the current disclosure have experimentally proved that effective tissue cooling supports increase in treatment energy, shortens treatment time and leads to better treatment results.

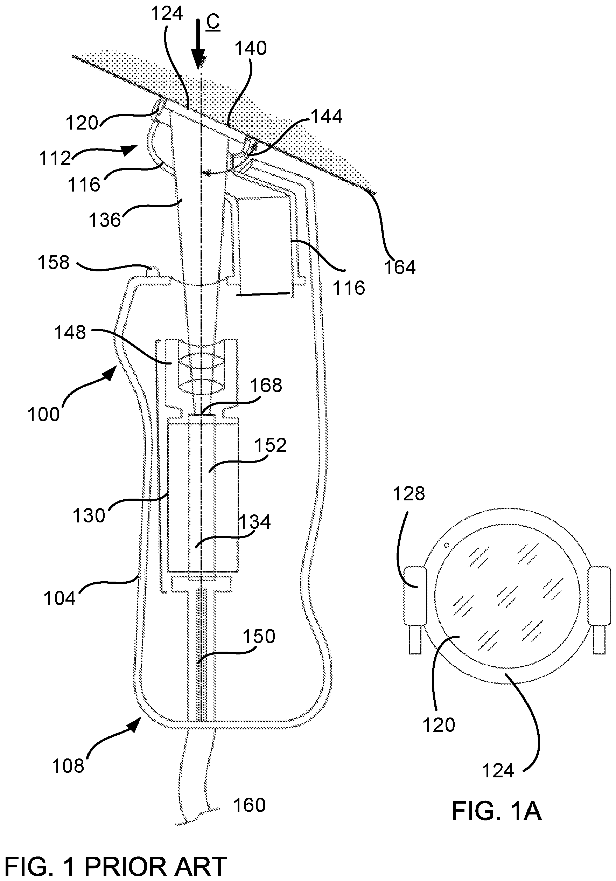

FIG. 1 is a simplified illustration of an existing an earlier disclosed applicator. Applicator or handpiece 100 includes a housing 104 with a proximal 108 and a distal 112 end. A support structure 116 including two support columns is terminated by a frame 120 that extends from distal end 112. Support structure 116 is angled to applicator 100 axis of symmetry 134 or off normal relative to axis of symmetry 134 of applicator 100 and improves line of sight to the skin treatment plane or skin area to be treated 140. The compliment of angle 144 at which support structure 116 is angled could be 10 to 30 degrees and usually the angle could be about 15 degrees.

Frame 120 holds an easy removable and replaceable window 124. Frame 120 could have a round, elliptical or a rectangular shape. Support structure 116 and frame 120 are made of metal or other material supporting good heat or cold conducting properties. Support structure 116 and frame 120 are in thermal communication with a thermoelectric cooler 118. Replaceable window 124 could be made of sapphire or quartz.

Applicator 100 further includes an optical system 130 configured to receive a beam of optical energy 136 from a source of optical energy (not shown) and to direct the beam of optical energy 136 to irradiate the skin or tissue treatment plane 140 defined by frame 120 and being in contact with replaceable window 124 in the tissue or skin treatment plane 140. Optical system 130 of applicator 100 could further include a pair of lenses 148 configured to receive a homogenized beam of optical energy 134 and image the output facet 168 of homogenizing rod 152 to the tissue or skin treatment plane 140.

In use applicator 100 is applied to the skin or tissue treatment plane 140 and the source of optical energy could be activated to apply treatment energy to the skin or tissue treatment plane 140. Replaceable window 124 is cooled through the contact with frame 120 and being in contact with the tissue or skin treatment plane 140, windows 124 cools the skin surface located in the tissue treatment plane 140.

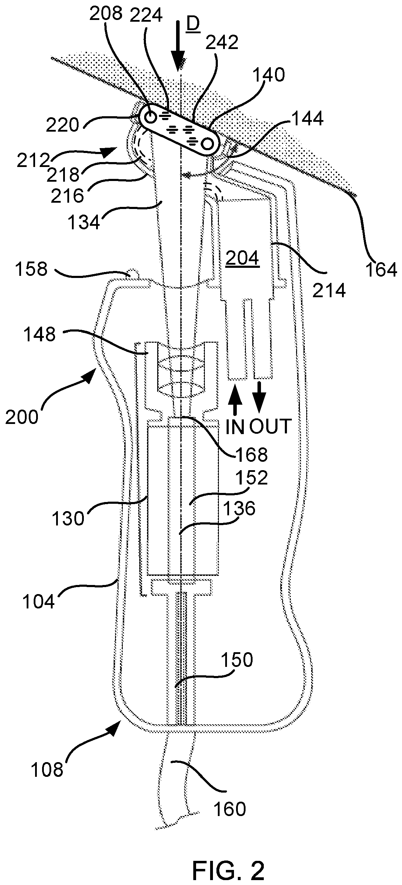

A cable 160 (FIG. 1 and FIG. 2) extends from proximal end 108 of applicator 100 housing 104. Cable 160 connects applicator 100 to a power supply (not shown). Housing of the power supply could also incorporate a source of optical energy and a source of RF voltage. Optical fiber guide 150 could be included in cable 160. The fiber could conduct the optical energy to applicator or handpiece 100/200/700. Alternatively, an articulated arm could conduct the optical energy to handpiece. Although not inclusive, the source of optical energy could be a suitable laser, for example, a 595 nm pulse dye laser, a 1064 nm Nd:YAG laser, or Alexandrite 755 nm laser, or a diode laser or an assembly of diode lasers with different laser diodes emitting different light wavelengths. Selection of laser depends on the desired type of treatment. The source of optical energy could be a suitable laser that provides optical energy with wavelength of 400nm to 2000nm. Since some of the treatment could be performed by wavelengths that are not visible, applicator 100 could also include a LED 158 illuminating the located between RF electrodes tissue or skin treatment area 140.

FIG. 1A, is a view of the applicator 100 distal end 112 as viewed from the direction indicated by arrow C (FIG. 1). A pair of bipolar RF electrodes 128 could be located on distal end 112 of housing 104. Electrodes 128 could be configured to apply RF energy to tissue 164 located in tissue treatment plane 140. Electrodes 128 could be solid metal electrodes mounted on frame 120 or deposited directly to the transparent window 120. Optical system 130 could be configured to receive a homogenized beam of optical energy 134 from light pipe homogenizing rod 152 and image output facet 168 of light pipe homogenizing rod 130 to a treated skin or tissue plane 140 located between the pair of bipolar RF electrodes 128.

Depending on the desired skin treatment, type of the skin, intensity of the energy applied to the skin or tissue treatment plane 140 the cooling by cooling plate 124 could be not sufficient and still cause an epidermal injury. The effectivity of the contact cooling devices could be further optimized to make them just as effective for both the lighter and darker skin types.

FIG. 2 is a simplified illustration of an applicator according to an example. Support structure 216 of applicator 200 includes two support columns 214 that extend from distal end 212 of handpiece or applicator 200.

Support structure 216 holds a transparent to treatment radiation window 224. Support structure 216 could include a slot 220 of rectangular or oval shape. Slot 220 is adapted to receive transparent window 224 made of sapphire or quartz. Support structure 216 is made of metal or other material supporting good heat or cold conducting properties, and RF electrical conducting properties. Support structure 216 includes one or more cooling fluid conducting channels 218 (shown in broken line.) receiving a cooling fluid from a cooled or chilled reservoir 204. Cooling fluid conducting channels 208 made in transparent window 224 are in fluid communication with fluid conducting channels 218 (shown in broken lines) made in the support structure 216. Fluid conducting channels 218 made in the support structure 216 and are in fluid communication with a reservoir 204 of cooling fluid. The flow of cooling fluid into the support structure and transparent window 224 with fluid conducting channels 208 is schematically shown by arrows IN and OUT. The fluid conducting channels 208 could be configured to conduct the cooling fluid to almost the entire surface of the transparent window 224 or at least to a part of the surface of the transparent window. A thermoelectric chiller or vapor compressor chiller (not shown) could be used to cool and maintain at a desired temperature cooling fluid in the reservoir 204.

Applicator 200 further includes elements similar or identical to the ones included in applicator 100. These elements are marked by identical referral numbers. An optical system 130 configured to receive a beam of optical energy 134 from a source of optical energy (not shown) directs the beam of optical energy 134 to irradiate the skin or tissue treatment plane 140 defined by transparent window 224. Surface 242 of the outer side of the transparent window is in contact with the tissue or skin treatment plane 140. Beam of optical energy 134 could be conducted from a source of optical energy (not shown) for example, by a fiber optics guide.

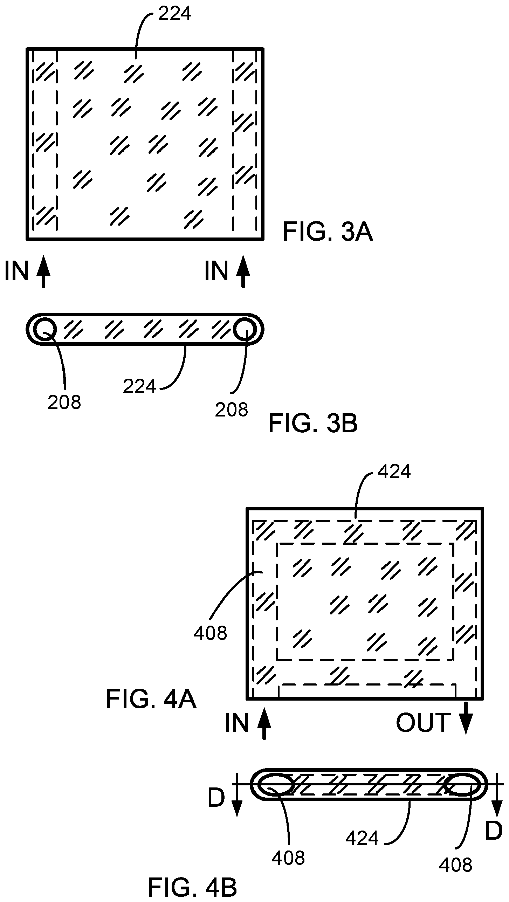

FIG. 3A and 3B is an example of a transparent window including cooling fluid conducting channels. Transparent window 224 (FIG. 3A) with cooling fluid conducting channels 208 delivers cooled or chilled fluid that could be water directly to and through the sapphire window 224 placed in contact with skin (see FIG. 2.) The proximity of the cooled or chilled water with the skin surface reduces the thermal resistance of the chilling/cooling tip. The thermal resistance just under 1 K/W is easily obtained by optimization of the chilled tip design. (In comparison for the thermoelectric cooler (TEC) cooled chilled tip designs, thermal resistances are typically around 8 K/W.)

In one example, transparent window 224 could include one or more separate cooling fluid conducting channels 208. The support structure 216 (FIG. 2) could be constructed to support flow of the cooling fluid in the same direction. In general, the fluid conducting channels 208 (FIG. 3B) are positioned near to but outside of the transmitted optical path to avoid lensing affects at the interface due to unmatched refractive indices between the fluid and transparent window material.

FIG. 4A and 4B is an additional example of a transparent window including cooling fluid conducting channels. Fluid conducting channels 408 are located along the perimeter of transparent sapphire window 424. In this example all four sides of the sapphire window 424 are in direct contact with the flowing cooled or chilled water. The four sided sapphire window cooling significantly reduces thermal gradients in the sapphire window. The cross section of the fluid conducting channels 408 (FIG. 4B) could be round elliptical, oval or any other cross section having a relatively low fluid flow resistance.

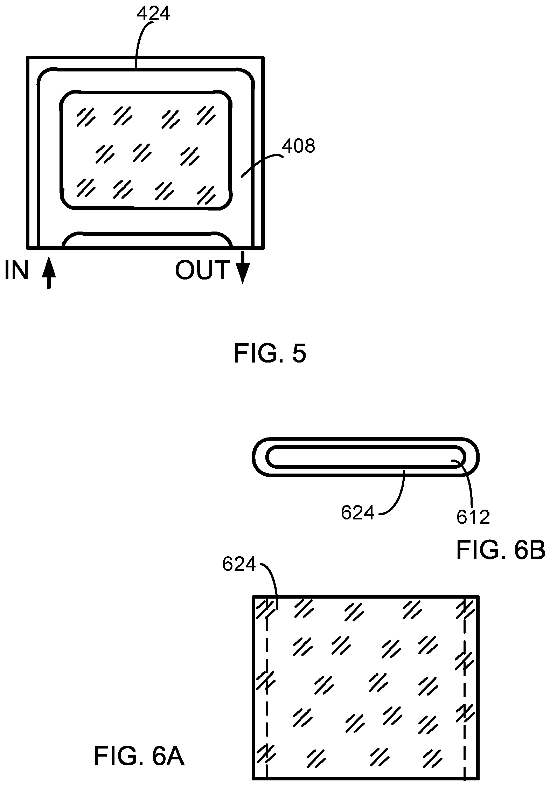

FIG. 5 is a cross section of cooling window 424 along line indicated by arrows D-D. The figure illustrates the geometry and rounded corners of fluid conducting channels 408. One of more fluid conducting channel can be positioned within the optical path although care should be taken that the cooling fluid and optical window material are selected to have similar refractive indices.

In another example, illustrated in FIG. 6A and 6B transparent window 624 could include a hollow cavity 612 being in fluid communication with fluid flow conducting channels 218 made in the support structure 216. In cases where the transparent window 624 has large size, for example 40 mm or 50 mm transparent window with hollow cavity 612 could become more effective avoiding the need for multiple channels. Although, to avoid optical reflective losses, the refractive indices of the window and cooling fluid should be similar.

The transparent window 624 having a hollow cavity 612 (and other examples of the transparent windows 224, 424 and 624) can be made from a single window material piece or could be made from two separate window material pieces that are held together with a support structure using a spacer to form a gap between the windows for coolant flow. Each surface of the transparent window or windows can have anti-reflective coatings designed to minimize optical reflective losses.

Support structures 116 and 216 illustrated in FIG. 1 and FIG. 2 are angled support structure. Support structure can also be design such that it approaches the tissue treatment plane 140 from the top or bottom of the applicator 100 and 200 to avoid getting in the way of the users direct line of site of the tissue treatment plane 140. The support structure can also be design such that it has an opening or void to avoid getting in the way of the users direct line of site of the tissue treatment plane 140.

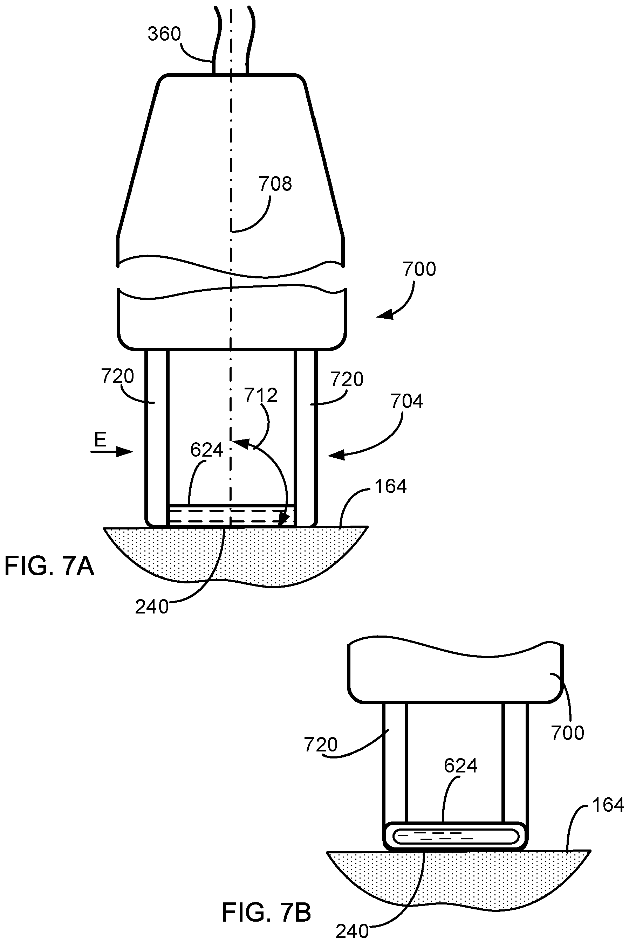

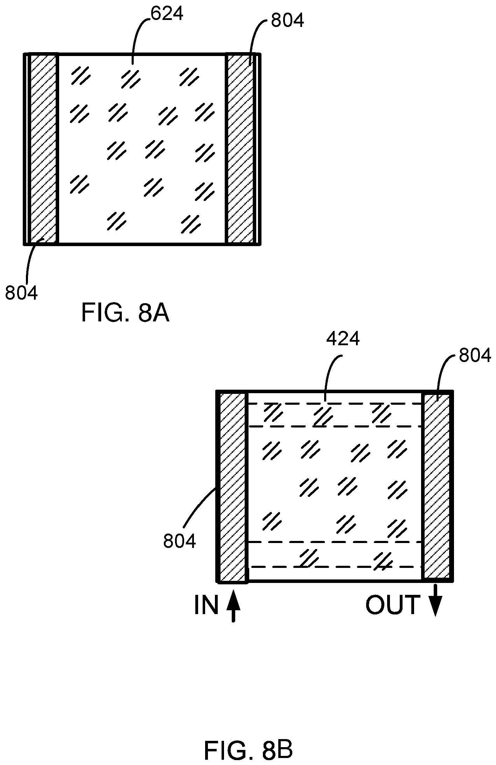

In another example illustrated in FIG. 7A and 7B, support structure 704 is oriented generally coaxial or the axis of the support structure 704 coincides with the axis of symmetry 708 of applicator 700 and is perpendicular to the tissue treatment plane 240 (Angle 712). The support structure is an assembly of cooling fluid conducting tubes 720 or channels. The open space between the cooling fluid conducting tubes 720 that could be made of rigid material is sufficient to support unobstructed real time observation or visualization of the tissue treatment plane during cosmetic skin treatment procedures. The distal end of the support structure 704 is configured to support or hold for example a transparent window 624 (FIG. 7B) or any other window with cooling fluid conducting channels. In some examples, as illustrated in FIG. 8A and 8B, a pair of bipolar RF electrodes could be deposited on transparent window 624 (or any other window, such as window 224 or 424) or support structure 704. The tissue treatment plane is located between the bipolar RF electrodes. The optical system of applicator 700 has no angled parts and the image of output facet 168 of light pipe homogenizing rod 152 could be of round, rectangular or even polygonal shape at the tissue treatment plane 140.

The optical energy across the tissue treatment plane 240 is homogenized. When applicator 200 or 700 is moved from one skin treatment location to another skin treatment location, the rectangular image or spot could be densely packed in the tissue or skin 164 (FIGS. 1 and 2).

In use applicator 200 or700is applied to the skin or tissue treatment plane and the source of optical energy is activated. Then, in order to treat an adjacent skin surface area the applicator is either continuously displaced over the skin or stepped from one skin segment to the other. The rectangular shape of the treatment optical energy spot does not force overlap between the stepped spots and also does not leave non-overlapped and not treated skin areas or segments. The cooling fluid circulation could be continuous (before the treatment, in course of the treatment and after the treatment). Alternatively, the cooling fluid could be forced to circulate at the defined periods.

In critical skin treatment applications requiring effective skin cooling such as when treating the darker skin types V and VI, each of the applicators 100/200 and 700 could be configured to operate in a treatment mode that enforces a 200 to 300ms cool down time before delivering the laser energy. This mode is suitable for example when the applicator is used in a stamping mode and for the RF delivery tips as it measures the skin impedance via the RF electrodes. Once skin contact is detected from a change in impedance, the system controller (not shown) waits for 200 to 300ms before delivering the laser energy. Enforced cooling guarantees effective and user-consistent skin cooling with each delivered laser pulse.

As indicated the skin treatment system could be equipped by a number of light sources, for example, lasers to treat different skin conditions. For example, a 595nm pulse dye laser could be used to treat different vascular conditions, Alexandrite 755 nm laser could be used for hair removal, a 1064 nm Nd:YAG laser could also be effective in treatment of different vascular conditions, different laser diodes emitting different light wavelengths could be used to treat a variety of skin condition by changing the treatment optical energy wavelength and pulse duration.

The fluence of optical energy used for skin or tissue conditions treatment could be 0.5 to 150 J/cm2, pulse duration could be 100 psec to 1 sec, the spot size could be 10X10 mm or even 20X20mm. Other sizes and relations between the spot size parameters could be considered including a rectangle such as 10X30mm

The authors of the current disclosure are aware that some cosmetic skin treatment system claim use of square or rectangular beams produced by a square or rectangular optical prism that is placed in contact with skin by cooling the prism. Although the latter approach has the benefit of cooling the skin surface, but in order to enhance or collect the incident light the prism is covered by light reflecting coatings and obstructs the operator's field of view. Use of the present applicator provides effective cooling of the treated skin area and mitigates adverse effects caused by application to the skin of non-uniform distributed energies and in particular light energy.

The applicator or handpiece significantly improve observation of the tissue or skin treatment plane and facilitate the work of the operator or caregiver, providing unobstructed view of the tissue or skin treatment plane.

It should be recognized that a number of variations of the above-described examples will be obvious to one of ordinary skill. Accordingly, the apparatus and method are not to be limited by those specific examples and methods as shown and described herein. Rather, the scope of the product and method is to be defined by the following claims and their equivalents.

## Claims

1. An applicator (200) with a skin cooling arrangement, the applicator (200) comprising:
a housing (104) and a support structure (216) extending from a distal end (212) of the housing (104), the support structure (216) including at least one cooling fluid conducting channel (218);
a transparent window (224) mounted on the support structure (216), the transparent window (224) including at least one cooling fluid conducting channel (208) being in fluid communication with the at least one fluid conducting channel (218) in the support structure (216), the transparent window (224) arranged to cool a treated tissue area in contact with the transparent window (224);
a light pipe homogenizing rod (152); and **characterized by**:
a pair of lenses (148) configured to receive a homogenized beam of optical energy (134) from the light pipe homogenizing rod (152) and to image an output facet (168) of the light pipe homogenizing rod (152) to a treated skin or tissue plane (140),
wherein the output facet (168) of the light pipe homogenizing rod (152) has a trapezoidal or oval shape.

2. The applicator (200) according to claim 1, further comprising a pair of bipolar RF electrodes (128) mounted on the support structure (216) and configured to apply RF energy to tissue located in the treated skin or tissue plane (140).

3. The applicator (200) according to claim 2, comprising an optical system (130) configured to receive a beam of optical energy (136) from a source of optical energy and convey it to tissue located in the treated skin or tissue plane (140) located between the pair of bipolar RF electrodes (128).

4. The applicator (200) according to claim 1, wherein a cross section of the cooling fluid conducting channel (208) in the transparent window (224) is one of a group consisting of a round, elliptical and oval cross section.

5. The applicator (200) according to claim 1, wherein the transparent window (624) includes a hollow cavity (612) in fluid communication with fluid conducting channels (208) in the support structure (218).

6. The applicator (200) according to claim 1, wherein the transparent window (224) is made of at least one of a group of materials consisting of sapphire and quartz.

7. The applicator (200) according to claim 1, wherein an outer surface of the transparent window (224) defines the treated skin or tissue plane (140) when in contact with tissue.

8. The applicator (200) according to claim 1, comprising a source of optical energy configured to provide optical energy with a wavelength of 400nm to 2000nm.

9. The applicator (200) according to claim 1, comprising a source of optical energy configured to provide optical energy with a fluence of 0.5 J/cm² to 150 J/cm².

10. The applicator (700) according to claim 1, wherein the support structure (704) is coaxial with an axis of symmetry (708) of the applicator (700).

11. The applicator (200) according to claim 1 wherein a cooling tip has a thermal resistance of less than 1 degree Celsius per Watt of heat load.

12. The applicator (200) according to claim 1, wherein the light pipe homogenizing rod (152) is at least one of a group of light guides consisting of a tapered light homogenizing rod, a tapered optical trapezoidal prism, a tapered multi-facet optical homogenizing rod and a tapered hollow light guide with a reflective coating deposited on inner surfaces thereof.

13. The applicator (200) according to claim 1, wherein the support structure (216) is angled with respect to an axis of symmetry of the applicator (200) at an angle of 10 to 30 degrees to facilitate the operator a good view of a treated tissue area (140).

14. The applicator (200) according to claim 1, further comprising a cooling fluid reservoir (204) and a thermoelectric element configured to cool the cooling fluid reservoir (204), wherein the cooling fluid cools the support structure (216) and a sapphire window (224) in contact with the treated skin or tissue plane (140).

15. The applicator (200) according to claim 14, wherein a cooling fluid circulation is in at least one of a group consisting of continuous circulation before the tissue treatment, in course of the tissue treatment and after the tissue treatment, wherein the cooling fluid is forced to circulate at defined periods and wherein the forced cooling fluid circulation is configured to operate in at least 200ms before delivery of laser energy.

## Patentansprüche

1. Applikator (200) mit einer Hautkühlungsanordnung, wobei der Applikator (200) umfasst:
ein Gehäuse (104) und eine Trägerstruktur (216), die sich von einem distalen Ende (212) des Gehäuses (104) erstrecken, wobei die Stützstruktur (216) mindestens einen Kühlfluid-Leitungskanal (218) einschließt;
ein transparentes Fenster (224), das an der Trägerstruktur (216) angebracht ist, wobei das transparente Fenster (224) mindestens einen Kühlfluid-Leitungskanal (208) einschließt, der mit dem mindestens einen Fluidleitungskanal (218) in der Trägerstruktur (216) in Fluidverbindung steht, wobei das transparente Fenster (224) angeordnet ist, um einen behandelten Gewebebereich in Kontakt mit dem transparenten Fenster (224) zu kühlen;
einen Lichtleiter-Homogenisierungsstab (152); und **gekennzeichnet durch**:
ein Paar Linsen (148), das konfiguriert ist, um einen homogenisierten Strahl optischer Energie (134) von dem Lichtleiter-Homogenisierungsstab (152) zu empfangen und eine Ausgangsfacette (168) des Lichtleiter-Homogenisierungsstabes (152) auf eine behandelte Haut- oder Gewebeebene (140) abzubilden,
wobei die Ausgangsfacette (168) des Lichtleiter-Homogenisierungsstabes (152) eine trapezförmige oder ovale Form aufweist.

2. Applikator (200) nach Anspruch 1, weiter umfassend ein Paar bipolarer HF-Elektroden (128), das an der Trägerstruktur (216) angebracht ist und konfiguriert ist, um HF-Energie an Gewebe anzulegen, das sich in der behandelten Haut oder Gewebeebene (140) befindet.

3. Applikator (200) nach Anspruch 2, umfassend ein optisches System (130), das konfiguriert ist, um einen Strahl optischer Energie (136) von einer Quelle optischer Energie zu empfangen und zu Gewebe zu befördern, das sich in der behandelten Haut oder Gewebeebene (140) befindet, die sich zwischen dem Paar von bipolaren HF-Elektroden (128) befindet.

4. Applikator (200) nach Anspruch 1, wobei ein Querschnitt des Kühlfluid-Leitungskanals (208) in dem transparenten Fenster (224) eine Gruppe ist, die aus einem runden, elliptischen und ovalen Querschnitt besteht.

5. Applikator (200) nach Anspruch 1, wobei das transparente Fenster (624) einen Hohlraum (612) in Fluidverbindung mit Fluidleitungskanälen (208) in der Trägerstruktur (218) einschließt.

6. Applikator (200) nach Anspruch 1, wobei das transparente Fenster (224) aus mindestens einem Material aus einer Gruppe von Materialien hergestellt ist, die aus Saphir und Quarz besteht.

7. Applikator (200) nach Anspruch 1, wobei eine Außenoberfläche des transparenten Fensters (224) die behandelte Haut- oder Gewebeebene (140) definiert, wenn sie mit Gewebe in Kontakt steht.

8. Applikator (200) nach Anspruch 1, umfassend eine Quelle für optische Energie, die konfiguriert ist, um optische Energie mit einer Wellenlänge von 400 nm bis 2000 nm bereitzustellen.

9. Applikator (200) nach Anspruch 1, umfassend eine Quelle für optische Energie, die konfiguriert ist, um optische Energie mit einer Fluenz von 0,5 J/cm² bis 150 J/cm² bereitzustellen.

10. Applikator (700) nach Anspruch 1, wobei die Trägerstruktur (704) koaxial zu einer Symmetrieachse (708) des Applikators (700) ist.

11. Applikator (200) nach Anspruch 1, wobei eine Kühlspitze einen Wärmewiderstand von weniger als 1 Grad Celsius pro Watt Wärmelast aufweist.

12. Applikator (200) nach Anspruch 1, wobei der Lichtleiter-Homogenisierungsstab (152) mindestens einer aus einer Gruppe von Lichtleitern ist, die aus einem sich verjüngenden Lichthomogenisierungsstab, einem sich verjüngenden optischen Trapezprisma, einem sich verjüngenden optischen Homogenisierungsstab mit mehreren Facetten und einem sich verjüngenden hohlen Lichtleiter mit einer reflektierenden Beschichtung, die auf den Innenoberflächen davon abgeschieden ist, besteht.

13. Applikator (200) nach Anspruch 1, wobei die Trägerstruktur (216) in Bezug auf eine Symmetrieachse des Applikators (200) in einem Winkel von 10 bis 30 Grad abgewinkelt ist, um dem Bediener eine gute Sicht auf einen behandelten Gewebebereich (140) zu erleichtern.

14. Applikator (200) nach Anspruch 1, weiter umfassend ein Kühlfluidreservoir (204) und ein thermoelektrisches Element, das zum Kühlen des Kühlfluidreservoirs (204) konfiguriert ist, wobei das Kühlfluid die Trägerstruktur (216) und ein Saphirfenster (224) in Kontakt mit der behandelten Haut- oder Gewebeebene (140) kühlt.

15. Applikator (200) nach Anspruch 14, wobei ein Kühlfluidkreislauf vor der Gewebebehandlung, im Verlauf der Gewebebehandlung und nach der Gewebebehandlung in mindestens einer Gruppe, die aus einem kontinuierlichem Kreislauf besteht, ist, wobei das Kühlfluid gezwungen wird, in definierten Zeiträumen zu zirkulieren, und wobei der erzwungene Kühlfluidkreislauf so konfiguriert ist, um in mindestens 200 ms vor der Abgabe von Laserenergie zu arbeiten.

## Revendications

1. Applicateur (200) avec un agencement de refroidissement de la peau, l'applicateur (200) comprenant :
un boîtier (104) et une structure de support (216) s'étendant à partir d'une extrémité distale (212) du boîtier (104), la structure de support (216) incluant au moins un canal conducteur de fluide de refroidissement (218) ;
une fenêtre transparente (224) montée sur la structure de support (216), la fenêtre transparente (224) incluant au moins un canal conducteur de fluide de refroidissement (208) en communication fluidique avec le au moins un canal conducteur de fluide (218) dans la structure de support (216), la fenêtre transparente (224) étant agencée pour refroidir une zone de tissu traitée en contact avec la fenêtre transparente (224) ;
une tige d'homogénéisation de conduit de lumière (152) ; et **caractérisé par** :
une paire de lentilles (148) configurées pour recevoir un faisceau d'énergie optique homogénéisé (134) provenant de la tige d'homogénéisation de conduit de lumière (152) et pour imager une facette de sortie (168) de la tige d'homogénéisation de conduit de lumière (152) sur un plan de peau ou de tissu traité (140),
dans lequel la facette de sortie (168) de la tige d'homogénéisation de conduit de lumière (152) a une forme trapézoïdale ou ovale.

2. Applicateur (200) selon la revendication 1, comprenant en outre une paire d'électrodes RF bipolaires (128) montées sur la structure de support (216) et configurées pour appliquer de l'énergie RF au tissu situé dans le plan de peau ou de tissu traité (140).

3. Applicateur (200) selon la revendication 2, comprenant un système optique (130) configuré pour recevoir un faisceau d'énergie optique (136) à partir d'une source d'énergie optique et le transmettre à un tissu situé dans le plan de peau ou de tissu traité (140) situé entre la paire d'électrodes RF bipolaires (128).

4. Applicateur (200) selon la revendication 1, dans lequel une section transversale du canal conducteur de fluide de refroidissement (208) dans la fenêtre transparente (224) fait partie d'un groupe constitué d'une section transversale ronde, elliptique et ovale.

5. Applicateur (200) selon la revendication 1, dans lequel la fenêtre transparente (624) inclut une cavité creuse (612) en communication fluidique avec des canaux conducteurs de fluide (208) dans la structure de support (218).

6. Applicateur (200) selon la revendication 1, dans lequel la fenêtre transparente (224) est constituée d'au moins un d'un groupe de matériaux constitué du saphir et du quartz.

7. Applicateur (200) selon la revendication 1, dans lequel une surface extérieure de la fenêtre transparente (224) définit le plan de peau ou de tissu traité (140) lorsqu'elle est en contact avec un tissu.

8. Applicateur (200) selon la revendication 1, comprenant une source d'énergie optique configurée pour fournir de l'énergie optique avec une longueur d'onde de 400 nm à 2 000 nm.

9. Applicateur (200) selon la revendication 1, comprenant une source d'énergie optique configurée pour fournir de l'énergie optique avec une fluence de 0,5 J/cm² à 150 J/cm².

10. Applicateur (700) selon la revendication 1, dans lequel la structure de support (704) est coaxiale à un axe de symétrie (708) de l'applicateur (700).

11. Applicateur (200) selon la revendication 1, dans lequel une pointe de refroidissement a une résistance thermique inférieure à 1 degré Celsius par Watt de charge thermique.

12. Applicateur (200) selon la revendication 1, dans lequel la tige d'homogénéisation de conduit de lumière (152) est au moins l'un d'un groupe de guides de lumière consistant en une tige d'homogénéisation de lumière effilée, un prisme trapézoïdal optique effilé, une tige d'homogénéisation optique effilée à multiples facettes et un guide de lumière creux effilé avec un revêtement réfléchissant déposé sur des surfaces intérieures de celui-ci.

13. Applicateur (200) selon la revendication 1, dans lequel la structure de support (216) est inclinée par rapport à un axe de symétrie de l'applicateur (200) selon un angle compris entre 10 et 30 degrés afin de faciliter à l'opérateur une bonne vue d'une zone de tissu traitée (140).

14. Applicateur (200) selon la revendication 1, comprenant en outre un réservoir de fluide de refroidissement (204) et un élément thermoélectrique configuré pour refroidir le réservoir de fluide de refroidissement (204), dans lequel le fluide de refroidissement refroidit la structure de support (216) et une fenêtre de saphir (224) en contact avec le plan de peau ou de tissu traité (140).

15. Applicateur (200) selon la revendication 14, dans lequel une circulation de fluide de refroidissement est dans au moins un d'un groupe consistant en une circulation continue avant le traitement du tissu, au cours du traitement du tissu et après le traitement du tissu, dans lequel le fluide de refroidissement est forcé à circuler à des périodes définies et dans lequel la circulation de fluide de refroidissement forcée est configurée pour fonctionner pendant au moins 200 ms avant la délivrance d'énergie laser.
